# Europäisches Patentamt

# European Patent Office

# Office européen des brevets

(19)

(11) Veröffentlichungsnummer: **0 047 971**
**B1**

(12) **EUROPÄISCHE PATENTSCHRIFT**

(45) Veröffentlichungstag der Patentschrift:
12.08.87

(51) Int. Cl.⁴: **C 08 F 292/00,** C 08 F 2/44,
A 61 K 6/08

(21) Anmeldenummer: **81107067.1**

(22) Anmeldetag: **08.09.81**

(54) Mineralische Teilchen, Verfahren zu ihrer Herstellung und sie enthaltende Dentalmaterialien.

(30) Priorität: **12.09.80 DE 3034374**

(43) Veröffentlichungstag der Anmeldung:
**24.03.82 Patentblatt 82/12**

(45) Bekanntmachung des Hinweises auf die Patenterteilung:
**12.08.87 Patentblatt 87/33**

(84) Benannte Vertragsstaaten:
**AT BE CH DE FR GB IT LI LU NL SE**

(56) Entgegenhaltungen:
EP-A-0 011 190
CH-A-471 841
DE-A-2 348 806
FR-A-1 353 675
GB-A-1 103 852
GB-A-1 195 214
US-A-3 423 830
US-A-3 519 593
US-A-3 897 586
US-A-4 028 325

Die Akte enthält technische Angaben, die nach
dem Eingang der Anmeldung eingereicht wurden
und die nicht in dieser Patentschrift enthalten sind.

(73) Patentinhaber: **DMG - Dental- Material-
Gesellschaft mbH, Fangdieckstrasse 61, D-2000
Hamburg 53 (DE)**

(72) Erfinder: **Engelbrecht, Jürgen, Dr. Dipl.- Chem.,
Kanalstrasse 37, D-2000 Hamburg 76 (DE)**
Erfinder: **Michael, Günther, Dr. Dipl.- Chem.,
Edvard- Munck- Strasse 39, D-2000 Hamburg 74
(DE)**
Erfinder: **Lustgarten, Stewart J., 73 Dalton Road,
Holliston Ma 01746 (US)**
Erfinder: **Mühlbauer, Ernst, Dipl.- Kfm.,
Dörpfeldstieg 3, D-2000 Hamburg 52 (DE)**

(74) Vertreter: **Glawe, Delfs, Moll & Partner
Patentanwälte, Rothenbaumchaussee 58
Postfach 2570, D-2000 Hamburg 13 (DE)**

## Beschreibung

Verwendung einer Masse aus einem polymerisierbaren, Monomere und/oder Oligomere enthaltenden Acrylat zur Herstellung eines Dentalmaterials.

Die Erfindung betrifft die Verwendung einer Masse aus einem polymerisierbaren, Monomere und/oder Oligomere enthaltenden Acrylat, einem Radikalpolymerisationsinitiator und einem aus feinen mineralischen Teilchen bestehenden anorganischen Füllstoff, wobei die Teilchen eine mittlere Teilchengröße von 0,1 bis 20 μm und eine Kunststoffbeschichtung mit einer mittleren Dicke von 0,01 bis 3 μm aufweisen, gegebenenfalls zusammen mit silanisierten, unbeschichteten Teilchen, zur Herstellung eines Dentalmaterials.

Mineralische Füllstoffteilchen mit einer Mohs-Härte von max. 5 zur Verwendung als Füllstoffe für Dentalmaterialien sind unter anderem aus folgenden Vorveröffentlichungen bekannt: US-PS 4 020 557, US-PS 4 028 325, DE-OS 1 570 971, DE-OS 2 850 916 und DE-OS 2 850 918. Im allgemeinen handelt es sich bei diesenFüllstoffen um kristalline oder amorphe Silikate oder Phosphate, die auch als Gläser vorliegen können. Diese Füllstoffe werden verschiedensten in der Kälte, in der Hitze oder durch UV-Bestrahlung härtbaren Kunststoffsystemen, meist auf Acrylatbasis, zugesetzt. Geeignete Kunststoffmaterialien auf Basis der genannten Acrylate sind ebenfalls aus den vorgenannten Veröffentlichungen sowie aus der DE-OS 2 432 013 bekannt. Kunststoffbeschichtete Teilchen der eingangs genannten Art sind weiterhin aus der EP-A-0 011 190 bekannt. Dimensionierungsangaben für die Kunststoffbeschichtung werden dort jedoch nicht gemacht.

Es wurde nun gefunden, daß man zu Dentalwerkstoffen mit besonders günstigen Eigenschaften kommt, wenn man eine geeignete Korngrößenfraktion mineralischer Teilchen, die an sich aus dem vorgenannten Stand der Technik bekannt sind, mit einer Deschichtung aus einem kalt-, licht- oder hitzegehärtetem Kunststoff versieht und die so erhaltenen, kunststoffbeschichteten Teilchen als Füllstoff in den ebenfalls an sich bekannten Dentalmaterial-Systemen verwendet.

Die unter erfindungsgemäßer Verwendung der mineralischen Teilchen der Erfindung erhaltenen Dentalwerkstoffe zeigen insbesondere den Vorteil, daß sie vor der Aushärtung eine nichtklebrige Konsistenz haben und daher leicht modelliert werden können. Weiterhin zeichnen sie sich durch eine ausgezeichnete Polierbarkeit, eine große Abrasionsfestigkeit und Härte, einen geringen thermischen Ausdehnungskoeffizienten und geringe Polymerisationsschrumpfung aus. Weiterhin zeigen sie eine gute Stopfbarkeit; daher können sie insbesondere als Zahnfüllungsmaterial im molaren Bereich eingesetzt werden.Im übrigen eignen sich die unter erfindungsgemäßer Verwendung der mineralischen Teilchender Erfindung erhaltenen Dentalwerkstoffe zur Anwendung im gesamten Dentalbereich , so z. B. auch als Kronen und Brückenmaterial sowie zur Herstellung von künstlichen Zähnen.

Vorzugsweise weisen die mineralischen Teilchen eine Mohs-Härte von max. etwa 7, insbesondere 3,0 - 5,0 auf. Sie können aus Siliziumdioxyd, Alumosilikaten mit Raumnetzstruktur und/oder Gläsern bestehen. Bevorzugt sind insbesondere Barium enthaltende Gläser und/oder Natrium, Kalium und/oder Calzium enthaltende Zeolithmaterialien. Es können jedoch auch mineralische Materialien mit anderer Mohs-Härte und Struktur eingesetzt werden.

Die vorgenannten Zeolithmaterialien sind vorzugsweise kristallin; sie können jedoch auch eine amorphe Struktur aufweisen. Die Barium enthaltenden Gläser können hergestellt werden, indem man bei einer möglichst niedrigen Temperatur (1100° - 1300°C) und während einer möglichst kurzen Zeitspanne (etwa 1 Stunde), die zum Erhalt einer homogenen Schmelze erforderlich ist, Siliziumdioxyd, Bariumflorid, Aluminiumoxyd und Boroxyd zusammenschmilzt. Bevorzugt. ist z.B, eine Glasmischung der folgenden Zusammensetzung

$SiO_2$ 44 Teile (in Mol %)
$BaF_2$ 28 Teile (in Mol %)
$Al_2O_3$ 12 Teile (in Mol %)
$B_2O_3$ 16 Teile (in Mol %)

Die Gläser werden in üblicher Weise geschmolszen, abgekühlt und fein vermahlen.

Als Kunststoff kann jedes aus dem Stand der Technik härtbare Material zur Beschichtung der mineralischen Teilchen eingesetzt werden. Bevorzugt wird ein Kunststoffmaterial eingesetzt, das mit dem Harzbindemittel in dem Dentalwerkstoff verträglich ist. Im Hinblick auf eine gute Verbindung zwischen Beschichtung und Matrix kann es zweckmäßig sein, für die Beschichtung den gleichen Kunststoff zu verwenden, der auch in der Matrix enthalten ist. Das Harzbindemittel kann irgendein übliches polymeres Bindemittel auf der Basis von Acrylaten sein, wie sie für die Herstellung von Dentalwerkstoffen, die Monomere und Oligomere enthalten, bekannt sind. Als besonders bevorzugtes Monomer oder Oligomer zur Verwendung als Kunststoffbeschichtungmaterial wird Triethylenglycoldimethacrylat verwendet.

Die Erfindung gibt den Vorteil, daß das die mineralischen Teilchen unmittelbar als Beschichtung umgebende Kunststoffmaterial ohne Rücksicht auf die späteren Verarbeitungsbedingungen gewählt werden kann. So kann beispielsweise ein Werkstoff oder ein Härtungsverfahren ausgewählt werden, das eine besondere hohe Festigkeit der Verbindung zwischen der Beschichtung und den mineralischen Teilchen oder eine besonders hohe Härte der Beschichtung erlaubt. Durch geeignete Wahl der Beschichtungshärte läßt sich so ein abgestufter Härteübergang von den mineralischen Füllstoffpartikeln zur Matrix und damit besonders gute Eigenschaften des erzielten Kunststoff-Formkörpers erreichen.

Es ist weiterhin vorteilhaft, ein Silanisierungsmittel wie Methacryloxypropyltrimethoxysilan einzusetzen, um die Adhäsion zwischen Bindemittel und Füllstoff zu verbessern; diese Maßnahme ist aus dem Stand der Technik bekannt.

Der Vorteil einer besseren Modellierbarkeit der Masse (Paste) ist nicht daran gebunden, daß ausschließlich beschichtete mineralische Teilchen als Füllstoff verwendet werden. Es kann vielmehr ein mehr Form eingesetzt werden. Als zweckmäßig haben sich Mischungsverhältnisse von 1:10 bis 10:1, insbesondere 1:5 bis 5:1 Gewichtsteilen erwiesen; besonders bevorzugt ist ein Mischungsverhältnis von 1:1.

Die Herstellung der mineralischen Teilchen kann in der Weise erfolgen, daß eine geeignete Teilchenfraktion mit einer Lösung eines Monomeren bzw. Oligomeren in einem geeigneten organischen Lösungsmittel behandelt wird. Z.B. können die Teilchen mit einer Lösung von Triethylenglycoldimethacrylat in Methylenchlorid behandelt werden. Nach der Behandlung wird das Gemisch eingedampft, anschließend werden die Monomeren in der Hitze polymerisiert. Das so erhaltene Gemisch kann vermahlen und anschließend zu einer geeigneten Teilchengröße, z. B. zwischen 1 und 10 µm, gesiebt werden. Die bei dieser Maßnahme möglicherweise unvermeidbare Aglomeration der Teilchen und die dadurch notwendig werdende Mahlung kann vermieden werden, wenn die Polymerisation in flüssiger Phase oder in gasförmiger Phase (Wirbelbett) vorgenommen wird.

Die beschichteten Füllstoffteilchen werden dann, ggf. zusammen mit unbeschichteten Teilchen, in üblicher Weise zu einem Dentalmaterial gegeben, worin sie dort die Hauptkomponente, gewöhnlich in einer Menge von mindestens 60 Gew.-% bilden. Dabei werden die unbeschichteten Teilchen vorzugsweise mit einem Silanisierungsmittel behandelt, um die Adhäsion zu steigern und die Teilchen zu hydrophobieren. Das Dentalmaterial kann in Form eines Paste/Paste - oder Paste/Flüssigkeitssystems abgefüllt werden; derartige Systeme sind bekannt. Das Dentalmaterial kann jedoch auch in Form eines eine einzige Paste umfassenden Systems formuliert werden, wobei das System einen üblichen Lichtinitiator oder mehrere für die Polymerisation im UV- oder sichtbaren Licht enthält. Die Konsistenz der Paste und die Formbarkeit kann durch den eingesetzten Anteil an beschichteten anorganischen Füllstoffteilchen gesteuert werden.

## Beispiel 1

Herstellung von mit polymerisiertem Urethandimethacrylat beschichteten Bariumglasteilchen:

340 g Bariumglas der mittleren Korngröße 5 - 10 µm werden auf übliche Weise mit Methacryloxypropyltrimethoxysilan silanisiert und mit 55 g 7,7,9-Tri-methyl-4,13.dioxo-3,14-dioxa-5,12-diazahexadecan-1,16-diol-dimethacrylat, 1,6 g Lauroylperoxid sowie mit 20 g hydrophobiertem amorphem Siliciumdioxid und 200 ml Methylenchlorid vermischt. Nach Eindampfen bis zur breiigen Konsistenz wird langsam in Wasser von 80°C eingerührt. Das ausfallende, das Bariumglas enthaltende Polymerisat wird abfiltriert, getrocknet, gemahlen und nach Korngrößen fraktioniert.

## Beispiel 2

Herstellung von mit polymerisierten Triethylenqlycoldimethacrylat beschichteten Zeolithteilchen:

200 g Natriumzeolith der mittleren Korngröße zwischen 1-4 µ werden auf übliche Weise mit Methacryloxypropyltrimethoxysilan silanisiert. Zusammen mit 50 g Triethylenglykoldimethacrylat, 8 g Azobisisobutyronitril und 200 ml Methylenchlorid wird bis zur lockeren Konsistenz gerührt und eingedampft.

Die Polymeriation wird bei 100°C vorgenommen. Der so entstandene Werkstoff wird gemahlen und in die gewünschten Korngrößenbereiche fraktioniert.

## Beispiel 3

Herstellung eines Dentalwerkstoffes:
a) Peroxidpaste:
300 g Polymerisat aus Beispiel 1 (Korngröße 1-15 µm)
33 g hydrophobiertes amorphes Siliciumdioxid
43 g Triethylenglycoldimethacrylat
35 g 2,2-bis[4-(3- methacryloxy-2-hydroxypropoxy)phenyl] -propan
61 g 2-Ethyl-2-hydroxymethyl-propandiol(1,3)-trimethacrylat
3,6 g Benzoylperoxid
Die einzelnen Komponenten werden intensiv miteinander verknetet und danach evakuiert.
b) Aminpaste
Polymerisat aus Beispiel 1 und alle anderen Bestangteile werden in gleichen Mengen wie bei der Peroxid-Paste a) eingesetzt und verarbeitet. Anstelle des Peroxids werden jedoch 3,6 g NN-Bis(2-hydroxypropyl)-3,5-dimethylanilin eingesetzt.
c) Dentalmasse
Gleiche Teile von Paste A und Paste B werden 30 Sekunden lang intensiv vermischt. Die erhaltene Mischung ist in hervorragender Weise als Zahnfüllungsmaterial geeignet. Sie härtet in wenigen Minuten unter sehr geringer Polymerisationsschrumpfung aus und kann anschließend glänzend poliert werden.

**Beispiel 4**

Herstellung eines Dentalwerkstoffs:
Entsprechend der Arbeitsweise aus Beispiel 3 werden aus
154 g Polymerisat aus Beispiel 2
280 g Natriumzeolith (Korngrößenbereich 1-9 µm, silanisiert mit Methacryloxypropyltrimethoxysilan)
33 g hydrophobiertes amorphes Siliciumdioxid
43 g Triethylenglycoldimethacrylat
35 g 2,2-Bis[4-(3-Methacryloxy-2-hydroxypropoxy)-phenyl]-propan
61 g 2-Ethyl-2-hydroxymethyl-propandiol(1,3)-trimethacrylat
3,6 g Benzoylperoxid bzw. 3,0 g N,N-Bis-(2-hydroxyethyl)p-toluidin eine Peroxid- und eine Aminpaste hergestellt. Beide Pasten werden wie das Material aus Beispiel 3 gemischt. Man erhält ebenfalls eine Mischung, die in hervorragender Weise als Zahnfüllungs material, und zwar besonders zum Stopfen von molaren Kavitäten geeignet ist.

Die die erfindungsgemäßen mineralischen Teilchen enthaltenden Dentalwerkstoffe zeichnen sich bei hohem Füllstoffgehalt durch geringe Polymerisationskontraktion, geringen thermischen Ausdehnungskoeffizienten, gute Polierbarkeit und große Abrasiofsresistenz aus.

**Patentansprüche**

1. Verwendung einer Masse aus einem polymerisierbaren, Monomere und/oder Oligomere enthaltenden Acrylat, einem Radikalpolymerisationsinitiator und einem aus feinen mineralischen Teilchen bestehenden anorganischen Füllstoff, wobei die Teilchen eine mittlere Teilchengröße von 0,1 bis 20 µm und eine Kunststoffbeschichtung mit einer mittleren Dicke von 0,01 bis 3 µm aufweisen, gegebenenfalls zusammen mit silanisierten, unbeschichteten Teilchen, zur Herstellung eines Dentalmaterials.

2. Verwendung nach Anspruch 1, dadurch gekennzeichnet, daß das Dentalmaterial einen Füllstoffanteil von mindestens 60 Gew.-% aufweist.

3. Verwendung nach Anspruch 1 oder 2, dadurch gekennzeichnet, daß die feinen Teilchen aus Siliciumdioxid, Alumosilikaten mit Raumnetzstruktur und/oder Gläsern bestehen.

4. Verwendung nach Anspruch 1 oder 2, dadurch gekennzeichnet, daß die Teilchen aus Barium enthaltenden Gläsern und/oder Natrium, Kalium und/oder Calzium enthaltenden Zeolithmaterialien bestehen.

5. Verwendung nach einem der Ansprüche 1 bis 4, dadurch gekennzeichnet, daß die Teilchen eine mittlere Größe von 0,1 bis 5 µm aufweisen.

6. Verwendung nach einem der Ansprüche 1 bis 5, dadurch gekennzeichnet, daß das Gewichtsverhältnis der beschichteten mineralischen Teilchen zu den unbeschichteten mineralischen Teilchen in dem Dentalmaterial 1:10 bis 10:1, insbesondere 1:5 bis 5:1, vorzugsweise 1:1, beträgt.

**Claims**

1. Use of a material consisting of a polymerisable acrylate containing monomers and/or oligomers, a radical polymerisation initiator and an inorganic filler consisting of fine mineral particles, the particles having an average thickness of 0.01 to 3 µm, possibly together with silanised, uncoated particles, for producing a dental material.

2. Use according to Claim 1, characterised in that the dental material comprises a proportion of filler of at least 60 % by weight.

3. Use according to Claim 1 or 2, characterised in that the fine particles consist of silicone dioxide, alumo-silicates with a spatial cellular structure and/or glasses.

4. Use according to Claim 1 or 2, characterised in that the particles consist of glasses containing barium and/or zeolite materials containing sodium, potassium and/or calcium.

5. Use according to one of Claims 1 to 4, characterised in that the particles have an average size of 0.1 to 5 µm.

6. Use according to one of Claims 1 to 5, characterised in that the weight ratio of the coated mineral particles to the uncoated mineral particles in the dental material amounts to 1:10 to 1:10, in particular 1:5 to 5:1, preferably 1:1.

**Revendications**

1. Utilisatian d'une matière se composant d'un acrylate polymérisable, contenant des manomères et/au des oligomères, d'un initiateur de polymérisation radicalaire et d'une matière de charpe inorganique composée de fines particules minérales, les particules présentant une grosseur moyenne de particules de 0,1 à 20 µm et un enduit de matière plastique d'une épaisseur moyenne de 0,01 à 3 µm, le cas échéant avec des particules silanisées non enduites, pour la preparation d'une matière à usage dentaire.

2. Utilisation selon la revendication 1, caractérisée en ce que la matière à usage dentaire presente une proportion de matière de charge d'au moins 60 % en poids.

3. Utilisation selon la revendication 1 ou 2, caractérisée en ce que les fines particules sont faites de bioxyde qe silicium, d'aluminosilicates à structure réticulee dans l'espace et/ou de verres.

4. Utilisation selon la revendication 1 ou 2, caractérisée en ce que les particules sont faites de verres contenant du baryum et/ou de matières zeolithiques contenant du sodium, du potassium et/ou du calcium.

5. Utilisation selon l'une quelconque des revendications 1 à 4, caractérisée en ce que les particules présentent une grosseur moyenne de 0,1 à 5 μm.

6. Utilisation selon l'une quelconque des revendications 1 à 5, caractérisée en ce que le rapport en poids des particules minérales enduites aux particules minérales non enduites dans la matière à usage dentaire se situe entre 1:10 et 10:1, en particulier entre 1:5 et 5:1, de préference à 1:1.